# EUROPÄISCHE PATENTSCHRIFT

(11) **EP 2 533 787 B1**
(45) Veröffentlichungstag und Bekanntmachung des Hinweises auf die Patenterteilung: **25.03.2015**
(21) Anmeldenummer: 11702254.1
(22) Anmeldetag: 08.02.2011
(51) Int. Cl.: A61K 31/7032, A61P 25/00

(54) **VERWENDUNG VON ISORHAMNETINTRIGLYCOSIDEN**
USE OF ISORHAMNETIN TRIGLYCOSIDES
UTILISATION DE TRIGLYCOSIDES D'ISORHAMNÉTINE

(30) Priorität: 11.02.2010 DE 102010001851
(43) Veröffentlichungstag der Anmeldung: 19.12.2012
(73) Patentinhaber: Dr. Willmar Schwabe GmbH & Co. KG, 76227 Karlsruhe (DE)
(72) Erfinder: NÖLDNER, Michael, 76139 Karlsruhe (DE); SCHÖTZ, Karl, 76448 Durmersheim (DE)
(74) Vertreter: Adam, Holger
(86) Internationale Anmeldenummer: PCT/EP2011/051821
(87) Internationale Veröffentlichungsnummer: WO 2011/098448

(56) Entgegenhaltungen:
- WO-A1-2004/078189
- WO-A1-2005/041994
- US-A- 6 004 560
- LOU H ET AL: "Alkaloids and flavonoids from peanut skins", MEDICINAL & AROMATIC PLANTS ABSTRACTS, SCIENTIFIC PUBLISHERS, SCIENTIFIC PUBLISHERS, NEW DELHI - INDIA, vol. 24, no. 1, 1 February 2002 (2002-02-01), XP018006663, ISSN: 0250-4367
- BECK M-A ET AL: "Flavonol glycosides from Eschscholtzia californica", PHYTOCHEMISTRY, PERGAMON PRESS, GB, vol. 50, no. 2, 26 January 1999 (1999-01-26), pages 329-332, XP004290730, ISSN: 0031-9422, DOI: 10.1016/S0031-9422(98)00503-2
- WICKENS JEFFERY R ET AL: "Animal models to guide clinical drug development in ADHD: lost in translation?", BRITISH JOURNAL OF PHARMACOLOGY, vol. 164, no. 4, Sp. Iss. SI, October 2011 (2011-10), pages 1107-1128,
- BLISS T V P ET AL: "A synaptic model of memory: Long-term potentiation in the hippocampus", NATURE (LONDON), vol. 361, no. 6407, 1993, pages 31-39, ISSN: 0028-0836
- MORRIS R: "DEVELOPMENTS OF A WATER MAZE PROCEDURE FOR STUDYING SPATIAL LEARNING IN THE RAT", JOURNAL OF NEUROSCIENCE METHODS, vol. 11, no. 1, 1984, pages 47-60, ISSN: 0165-0270

## Beschreibung

Die vorliegende Erfindung betrifft die Verwendung von Isorhamnetintriglycosiden sowie von Extrakten, die diese enthalten zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen sowie zur Verbesserung von Gedächtnisfunktionen. Ferner betrifft die Erfindung das Isorhamnetintriglycosid 2 sowie Pflanzenextrakte, die dieses Glycosid enthalten.

Die WO 2004/078189 offenbart die Verwendung von Rutin und Isorhamnetin zur Behandlung von depressiven Verstimmungen und Erkrankungen sowie bei anderen affektiven Störungen.

In der WO 2005/041994 A1 wird die Verwendung von Feigenkaktus (Opuntia), Pflanzenteilen davon und/oder von daraus hergestellten Extrakten oder sonstigen Zubereitungen zur Behandlung von depressiven Verstimmungen und Erkrankungen oder von anderen affektiven Störungen beschrieben.

Die US 6,004,560 beschreibt ein Nasenspray zur Behandlung von Fieber und Erkältungskrankheiten, umfassend 250-300 g Bupleurum Scorzonerifolium Willd, 120-144 g *Radix lastidis* Indigotica, 120-144 g *Folium lastidis*, und 10-12 g Vitamin C.

Lou H.; Yuan, H.; Yamazaki, Y.; Sasaki, T.; Oka, S., Planta Medica 67(4), Seiten 345-349, 2001 beschreiben Alkaloide und Flavonoide aus Erdnusshaut.

Mona-Antonia Beck und Hanns Häberlein in Phytochemistry 50 (1999) 329-332 beschreiben Flavonglycoside aus *Eschscholtzia californica.*

Neurologische Erkrankungen wie zum Beispiel das sogenannte "Aufmerksamkeits-Defizit-Hyperaktivitäts-Syndrom" (abgekürzt ADHS oder auch ADHD für "Attention Deficit Hyperactivity Disorder") werden mit großem Erfolg mit Arzneimitteln behandelt die selber motorisch stimulierend wirken z. B. Methylphenidat und Methamphetamin.

Darüber hinaus ist eine Vielzahl von anderen neurologischen und psychischen Erkrankungen von einer deutlichen Reduktion der Alltagsaktivitäten begleitet. So berichten depressive oder ängstliche Menschen häufig, dass sie Schwierigkeiten haben am normalen Erwerbsleben oder an Freizeitaktivitäten teilzunehmen. Dies führt zu Verlusten an sozialen Kontakten und zu einer immer weitergehenden Verschlechterung des Krankheitsbildes. Könnte durch eine medikamentöse Therapie dieses Vermeidungsverhalten positiv beeinflusst werden, könnte dies zu einer Steigerung der sozialen Kontakte, und damit zu einer Verbesserung des Krankheitsbildes führen. Damit könnten die bisher zur Behandlung derartiger psychischer Erkrankungen verwendeten Arzneimittel (Antidepressiva/Anxiolytika), die häufig zusätzlich sedierend wirken und damit die Inaktivität der Patienten steigern, wirksam unterstützt werden. Häufig sind gerade ältere Menschen, die bereits Defizite im mentalen Bereich aufweisen zusätzlich durch eine Reduktion der Alltagsaktivitäten betroffen, so dass eine Verbesserung sowohl der Aktivität, als auch der mentalen Funktionen zu einer Verbesserung der Lebensqualität der betroffenen Personen führen kann.

Aufgabe der vorliegenden Erfindung ist es somit ein Mittel bereitzustellen, das motivationsabhängiges Verhalten steigert und außerdem zu einer Verbesserung mentaler Funktionen führt und somit geeignet ist zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen sowie zur Verbesserung von Gedächtnisfunktionen.

Diese Aufgabe wird gelöst durch die Verwendung von Isorhamnetintriglycosiden der allgemeinen Formel **1** zur Herstellung eines Arzneimittels und eines Lebensmittels bzw. durch Isorhamnetintriglycosiden der allgemeinen Formel **1** als Arzneimittel und als Lebensmittel, 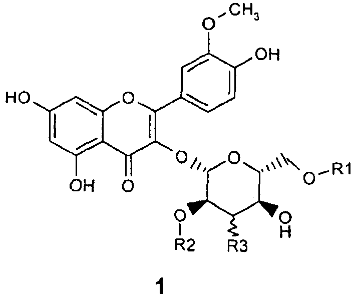 wobei in der Formel 1 R1 = α-L-Rha, R2 = ß-D-Xyl und R3 = 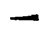 OH oder R¹ = α-L-Rha-(1→4)-α-L-Rha, R² = H und R³ = 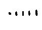 OH oder R¹ = α-L-Rha, R² = ß-D-Xyl und R³ = 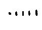 OH bedeuten, zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen, sowie zur Verbesserung von Gedächtnisfunktionen. Bevorzugt ist hierbei die Verwendung der Verbindungen 3-[(O-6-Deoxy-α-L-mannopyranosyl-(1→6)-O-[ß-D-xylopyranosyl-(1→2)]-ß-D-galactopyranosyl)oxy]-5,7-dihydroxy-2-(4-hydroxy-3-methoxy-phenyl)-4H-1-benzopyran-4-on (Verbindung **2,** R1 = α-L-Rha, R2 = ß-D-Xyl, R3 = 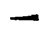 OH), 3-[(O-6-Deoxy-α-L-mannopyranosyl-(1→4)-O-6-deoxy-α-L-mannopyranosyl-(1→6)-ß-D-glucopyranosyl)oxy]-5,7-dihydroxy-2-(4-hydroxy-3-methoxyphenyl)-4H-1-benzopyran-4-on (Verbindung **3,** R1 = α-L-Rha-(1→4)-α-L-Rha, R2 = H, R3 = 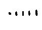 OH) und 3-[(O-6-Deoxy-α-L-mannopyranosyl-(1→6)-O-[ß-D-xylopyranosyl-(1→2)]-ß-D-glucopyranosyl)oxy]-5,7-dihydroxy-2-(4-hydroxy-3-methoxyphenyl)-4H-1-benzopyran-4-on (Verbindung **4,** R1 = α-L-Rha, R2 = ß-D-Xyl, R3 = 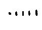 OH). Vorzugsweise ist die Verbindung der Formel 1 (insbesondere Verbindung 2, 3 und 4) Bestandteil eines Pflanzenextrakts (insbesondere eines Extraktes aus Blüten von Opuntia ficus-indica).

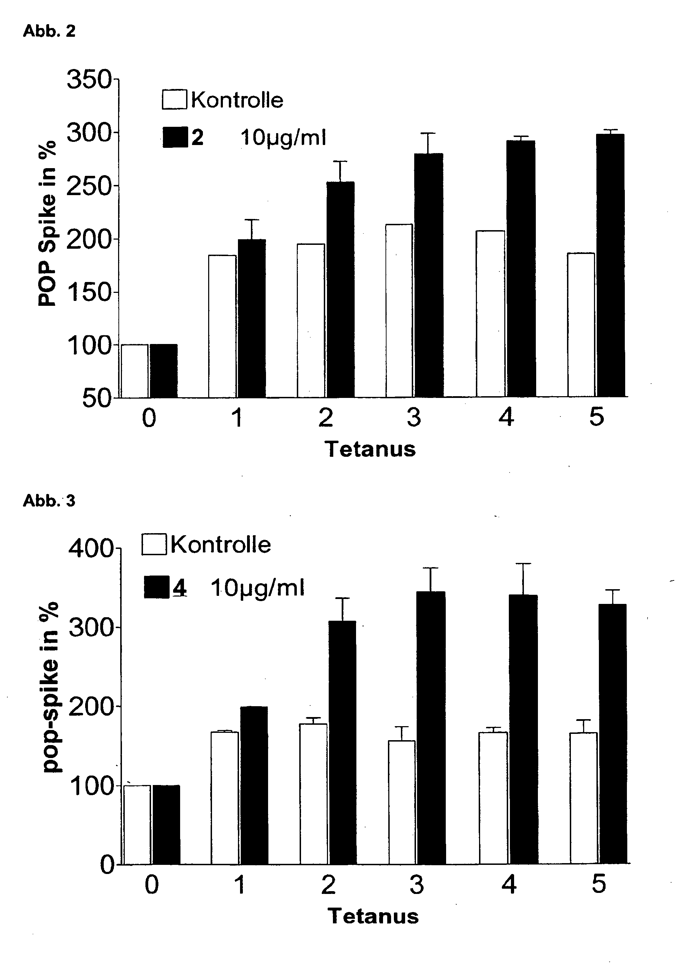

Es wurde nun überraschend festgestellt, dass die orale Applikation der Isorhamnetintriglycoside **2** und **3** bei Mäusen zu einem deutlichen, dosisabhängigen und statistisch signifikantem Ansteigen des Erkundungsverhaltens ("exploratory behavior") führt, ohne dass weitere Verhaltensparameter verändert werden (Tabelle 2). Außerdem wird die Langzeitpotenzierung in Ratten-Hirnschnitt-Präparaten (LTP-Modell) unter Einwirkung der Isorhamnetintriglycoside **2** und **4** deutlich gesteigert im Vergleich zu Kontrollexperimenten mit Pufferlösung (Abb. 2 und 3). Die Verbindungen der allgemeinen Formel 1 sind deshalb grundsätzlich geeignet zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, wie z. B. dem Aufmerksamkeits-Defizit-Hyperaktivitäts-Syndrom (ADHS), dem demenziellen Syndrom, Morbus Alzheimer, vaskuläre Demenzen und Gedächtnisstörungen anderer Ursachen. Darüber hinaus können die erfindungsgemäßen Isorhamnetintriglycoside zur Verbesserung von Gedächtnisfunktionen eingesetzt werden.

Ferner wurde festgestellt, dass ein Opuntienextrakt, der diese Substanzen enthält, in einem Tiermodell ("Morris Watermaze") eine signifikante Verbesserung der Lernleistung von Ratten, insbesondere des räumlichen Lernens zeigte.

Bei Verbindung **2** handelt sich um die bisher nicht beschriebene Substanz 3-[(O-6-Deoxy-α-L-mannopyranosyl-(1→6)-O-[ß-D-xylopyranosyl-(1→2)]-ß-D-galactopyranosyl)oxy]-5,7-dihydroxy-2-(4-hydroxy-3-methoxyphenyl)-4H-1-benzopyran-4-on (entsprechend 3-{α-L-Rha-(1→6)-[ß-D-Xyl-(1→2)]-ß-D-Gal}-isorhamnetin). Diese neue Verbindung **2** ist ebenfalls Gegenstand der vorliegenden Erfindung. Darüber hinaus sind auch Pflanzenextrakte, welche das Isorhamnetintriglycosid **2** enthalten, Gegenstand der Erfindung. Bevorzugt sind dabei Extrakte aus Opuntia-Arten und besonders bevorzugt aus Blüten von Opuntia ficus-indica.

Gegenstand der Erfindung sind darüber hinaus Arzneimittel, Lebensmittel und sonstige Zubereitungen, die eines oder mehrere der Isorhamnetintriglycoside **2, 3** und **4** oder Pflanzenextrakte, welche eines oder mehrere Isorhamnetintriglycoside **2**, **3** und **4** ggf. in Kombination mit anderen Stoffen, wie z. B. Wirk- und/oder Hilfsstoffen enthalten. Unter Lebensmittel sind hierbei insbesondere diätetische Lebensmittel, Nahrungsergänzungsmittel sowie "medical food" und "dietary supplements" zu verstehen.

Gegenstand der Erfindung sind insbesondere die nachstehenden Ausführungsformen:
1. Verwendung von Isorhamnetintriglycosiden der allgemeinen Formel **1,** 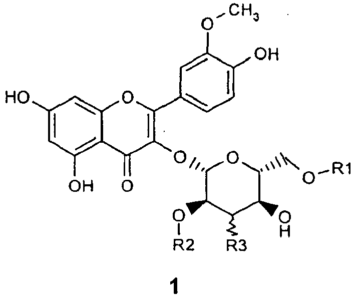
   worin R1 = α-L-Rha, R2 = ß-D-Xyl, R3 = 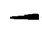 OH oder
   worin R1 = α-L-Rha-(1→4)-α-L-Rha, R2 = H, R3 = 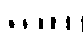 OH oder
   worin R1 = α-L-Rha, R2 = ß-D-Xyl, R3 = 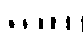 OH,
   zur Herstellung eines Arzneimittels zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen, sowie zur Verbesserung von Gedächtnisfunktionen.
2. Verwendung von Isorhamnetintriglycosiden der allgemeinen Formel **1,** 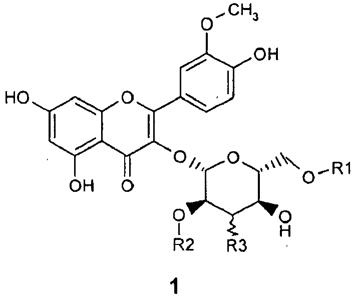
   worin R1 = α-L-Rha, R2 = ß-D-Xyl, R3 = 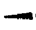 OH oder
   worin R1 = α-L-Rha-(1→4)-α-L-Rha, R2 = H, R3 = 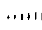 OH oder
   worin R1 = α-L-Rha, R2 = ß-D-Xyl, R3 = 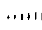 OH,
   zur Herstellung eines Lebensmittels oder als Lebensmittel zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen, sowie zur Verbesserung von Gedächtnisfunktionen.
3. Verbindung der allgemeinen Formel **1,** 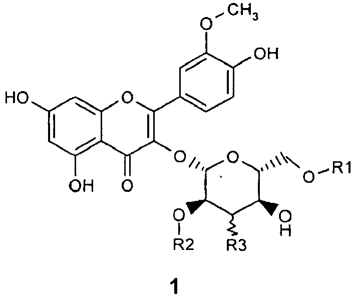
   worin R1 = α-L-Rha, R2 = ß-D-Xyl, R3 = 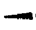 OH oder
   worin R1 = α-L-Rha-(1→4)-α-L-Rha, R2 = H, R3 = 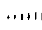 OH oder
   worin R1 = α-L-Rha, R2 = ß-D-Xyl, R3 = 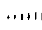 OH,
   zur Verwendung als Arzneimittel zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen, sowie zur Verbesserung von Gedächtnisfunktionen.
4. Verbindung der allgemeinen Formel **1,** 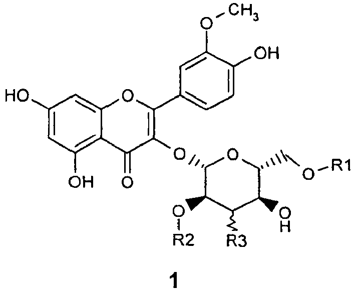
   worin R1 = α-L-Rha, R2 = ß-D-Xyl, R3 = 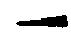 OH oder
   worin R1 = α-L-Rha-(1→4)-α-L-Rha, R2 = H, R3 = 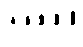 OH oder
   worin R1 = α-L-Rha, R2 = ß-D-Xyl, R3 = 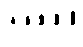 OH,
   zur Verwendung als Lebensmittel zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen, sowie zur Verbesserung von Gedächtnisfunktionen.
5. Verwendung bzw. Verbindung zur Verwendung nach einem der Absätze 1 bis 4, wobei eine oder mehrere der Verbindungen der allgemeinen Formel 1 in einem Pflanzenextrakt enthalten sind.
6. Verwendung bzw. Verbindung zur Verwendung nach Absatz 5, wobei der Pflanzenextrakt ein Extrakt aus Opuntien ist.
7. Verwendung bzw. Verbindung zur Verwendung nach Absatz 6, wobei der Pflanzenextrakt ein Extrakt aus Blüten von Opuntia ficus-indica ist.
8. Verwendung bzw. Verbindung zur Verwendung nach Absatz 7, wobei der Pflanzenextrakt aus Blüten von Opuntia ficus-indica mit 10 bis 90 Gew.-% Ethanol als Extraktionslösungsmittel hergestellt ist.
9. Verwendung bzw. Verbindung zur Verwendung nach Absatz 7, wobei der Pflanzenextrakt aus Blüten von Opuntia ficus-indica mit 50 bis 70 Gew.-% Ethanol als Extraktionslösungsmittel hergestellt ist.
10. Verwendung bzw. Verbindung zur Verwendung nach einem der Absätze 5 bis 9, wobei die Konzentration mindestens einer der Verbindungen der allgemeinen Formel **1** im Trockenanteil des Pflanzenextraktes im Bereich von 0,01 bis 10 Gew.-% liegt.
11. Verwendung bzw. Verbindung zur Verwendung nach Absatz 10, wobei die Konzentration mindestens einer der Verbindungen der allgemeinen Formel **1** im Trockenanteil des Pflanzenextraktes im Bereich von 0,05 bis 5% liegt.
12. Isorhamnetintriglycosid der allgemeinen Formel **1,** 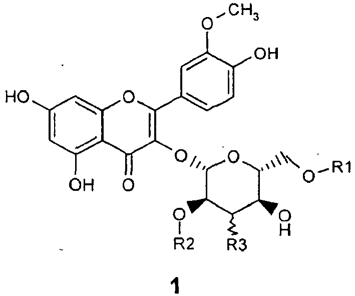 in der R1 = α-L-Rha, R2 = ß-D-Xyl und R3 = 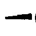 OH bedeuten (Verbindung 2).

- **Abb. 1**: Struktur von **2** mit wichtigen HMBC-Korrelationen
- **Abb. 2**: Einfluss von Isorhamnetintriglycosid **2** bzw. Kontrolllösung auf die Größe der POP Spikes nach wiederholter LTP-Induktion. Substanzzulauf beginnt 10 Minuten nach der 1. LTP-Induktion.
- **Abb. 3**: Einfluss von Isorhamnetintriglycosid **4** bzw. Kontrolllösung auf die Größe der POP Spikes nach wiederholter LTP-Induktion. Substanzzulauf beginnt 10 Minuten nach der 1. LTP-Induktion.
- **Abb. 4**: Häufigkeit des Findens der Plattform in Prozent an Tag 9 des Versuches gemäß Beispiel 5

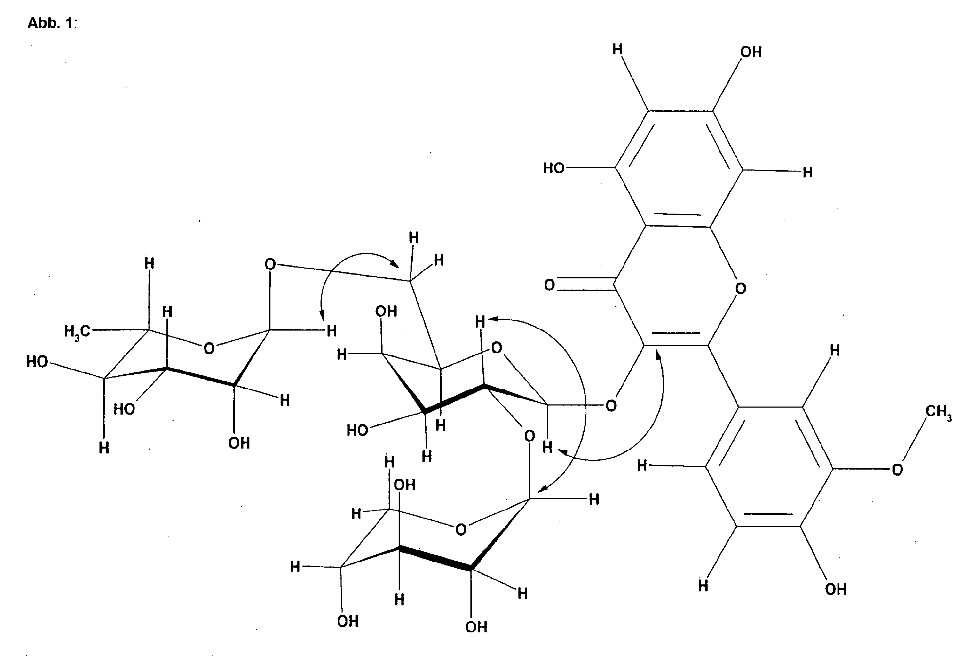

Die neue Verbindung **2** wurde aus einem Opuntien-Extrakt durch flüssig-flüssig-Verteilung, Säulenchromatographie und HPLC-Trennung als Reinsubstanz isoliert (Beispiel 2). Die Struktur ergab sich aus der Kombination von HSQCGP-Messungen, die eine eindeutige Zuordnung der Protonen zu den gebundenen Kohlenstoffatomen erlaubte. Darauf aufbauend konnte durch Anwendung von COSY und selektiver TOCSY-Messungen mit unterschiedlicher Mixing Time die Reihenfolge der Wasserstoffprotonen eines jeweiligen Zuckerrestes abgefragt und unter Berücksichtigung der Kopplungskonstanten eine nahezu eindeutige Zuordnung der jeweils drei Zuckereinheiten aufgebaut werden. Anschließend wurden durch HMBC-Messungen die Verknüpfung der Ringe untereinander, sowie die des Aglycons festgestellt (Abb. 1). Letztlich wurde der damit erhaltene Strukturvorschlag mittels ROESY auf Stimmigkeit überprüft (Tabelle 1). Bei den sehr ähnlichen chemischen Verschiebungen der Protonen H-2"' und H-3'" wurde der exakte Wert der Kopplungskonstanten und der chemischen Verschiebung durch Simulation des Kopplungsmusters im TOCSY-Spektrum abgeleitet. Zur Überprüfung der absoluten Konfiguration der Zuckerbausteine wurde eine saure Hydrolyse durchgeführt und die Monomeren mittels HPLC-MS und Vergleich mit Referenzverbindungen als D-Galaktose, D-Xylose und L-Rhamnose identifiziert.

Desgleichen wurden die Verbindungen **3** und **4** isoliert (Beispiel 2) und charakterisiert, wobei es sich um Derivate des Isoquercitrins handelt. Verbindung **3** wurde aus Eschscholtzia californica isoliert (M.-A. Beck und H. Häberlein, Phytochemistry 50 (1999), 329 - 332, dort Verbindung 6), jedoch nicht auf biologische Eigenschaften untersucht. Verbindung **4** wurde bereits in Erdnussschalen gefunden und dort auf Radikalfängereigenschaften und Antidiabetiswirkung untersucht (H. Lou et al., "Alkaloids and Flavonoids from Peanut Skins", Planta Med. 67 (2001) 345 - 349).

Die erfindungsgemäß verwendeten Extrakte aus Opuntia-Arten oder bevorzugt aus Blüten von Opuntia ficus-indica können nach an sich bekannten Herstellungsverfahren in variabler Zusammensetzung mit Lösungsmitteln wie z. B. Wasser, Methanol, Ethanol, Aceton, etc., und deren Gemische bei Temperaturen von Raumtemperatur bis 70 °C unter Durchmischung, d.h. durch Mazeration, innerhalb von 10 Min. bis 24 Std. oder alternativ durch Perkolation bei Raumtemperatur innerhalb von 1 Std. bis 24 Std. erhalten werden. Bevorzugte Extraktionslösungsmittel sind dabei Gemische von Ethanol und Wasser im Verhältnis Ethanol / Wasser = 10/90 bis 90/10 (w/w), besonders bevorzugt 50/50 bis 70/30 (w/w). Zur Herstellung eines Extraktes werden beispielsweise fein gemahlene getrocknete Blüten von Opuntia ficus-indica 2 mal mit der siebenfachen Gewichtsmenge 60 Gew.-% Ethanol 30 bis 120 Min. (z.B. 60 Min.) bei 50 bis 70 °C (z.B. 60 °C) extrahiert und die Lösungen filtriert. Anschließend werden die vereinigten Filtrate im Vakuum bei 40 °C weitgehend vom organischen Lösungsmittel befreit und die verbliebene Wasserphase getrocknet. Zur Anreicherung der erfindungsgemäßen Verbindungen **2, 3** und/oder **4** können weitere Konzentrierungsschritte durchgeführt werden wie z. B. flüssig-flüssig-Verteilung mit z. B. 1-Butanol/Wasser oder Ethylacetat/Wasser, Adsorption-Desorption an Ionenaustauscher, Sephadex LH20, Diaion HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck oder durch Gefriertrocknung. Trockenextrakte haben dabei gemäß dem Europäischen Arzneibuch im Allgemeinen einen Trockenrückstand von mindestens 95 Gew.-%.

Die erfindungsgemäß verwendeten Verbindungen der allgemeinen Formel **1** bzw. die mindestens eine dieser Verbindungen enthaltenden Extrakte können in Form von Pulvern, Granulaten, Tabletten, Dragees oder Kapseln oder auch als Lösung vorzugsweise oral verabreicht werden.

Die Dosierung erfolgt dabei so, dass pro Tag 0,1 mg bis 250 mg, vorzugsweise 0,3 mg bis 50 mg einer oder mehrerer der Verbindungen der allgemeinen Formel **1,** insbesondere einer oder mehrerer der Verbindungen 2, 3 und 4, zugeführt werden.

Zur Herstellung von Tabletten wird mindestens eine der Verbindungen der allgemeinen Formel 1, insbesondere einer oder mehrerer der Verbindungen 2, 3 und 4, bzw. der entsprechende Extrakt mit geeigneten pharmazeutisch verträglichen Hilfsstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

### Beispiele

### Beispiel 1: Herstellung eines Extraktes aus Blüten von Opuntia ficus-indica

500 g fein gemahlene getrocknete Blüten von Opuntia ficus-indica werden 2 mal mit der siebenfachen Gewichtsmenge 60 Gew.-% Ethanol 60 Min. bei 60 °C extrahiert und die Lösungen über einen Seitz Supra-Filter 1500 filtriert. Anschließend werden die vereinigten Filtrate i. Vak. bei 40 °C weitgehend vom org. Lösungsmittel befreit und die verbliebene Wasserphase gefriergetrocknet: 96,2 g (19,2 % bezogen auf das getrocknete Pflanzenmaterial). Der Extrakt enthält 0,10 % **2,** 0,13 % **3** und 0,24 % **4,.**

### Quantifizierung der Triglycoside 2 bis 4:

Zur Quantifizierung der Triglycoside wird der Extrakt in 50%igem MeOH aufgenommen (1 mg/ml), die Mischung filtriert und davon 10 ul (10.000 ng) für eine HPLC-MS-Quantifizierung eingesetzt. Der Gehalt wird mittels einer 4-Punkt-Eichung von 0.1 % bis 1% (10 bis 100 ng pro Injektion) der aufgereinigten Glycoside bestimmt.
HPLC-Bedingungen: Säule: Phenomenex Luna 5 u C18(2) (250x4,6 mm); Eluent A: MeCN/H₂O/HCOOH = 5:95:0,3, Eluent B: MeCN/H₂O/HCOOH = 59:41:0,3; Gradient: 8 Min. isokratisch 100% A, innerhalb von 22 Min. auf 50% A; Fluss: 0,6 ml/Min.
Zur Quantifizierung der LC-MS-Chromatogramme werden ausgewählte Massespuren der Pseudomolekülionen ausgewertet. Bei **3** wird m/z (M+H⁺) = 771 Da mit Rₜ = 5,4 Min., sowie bei **4** und **2** m/z (M+H⁺) = 757 Da mit Rₜ = 5.9 bzw. 6.2 Min. verwendet.

### Beispiel 2: Isolation der Triglycoside 2 bis 4

500,2 g 60 Gew.-%iger ethanolischer Extrakt aus Blüten von Opuntia ficus-indica (hergestellt gemäß Beispiel 1) werden mit 10 l H2O und 6,7 l Ethylacetat verteilt, die wässrige Phase filtriert, das Filtrat über ein Bett (d = 14 cm, h = 40 cm) von Diaion HP-20 gegeben und das Retentat mit Wasser eluiert. Das Wassereluat (11l) wird i. Vak. bei 40 °C weitgehend eingeengt und der Rückstand letztlich lyophilisert. Der verbliebene Feststoff (132,2 g) wird in 850 ml 50%igem MeOH aufgenommen und über eine Sephadex LH-20-Säule (d = 7,8 cm, h = 169 cm; ca. 50 ml pro Glas) mit 50%igem MeOH chromatographiert. Fraktionen ähnlicher Zusammensetzung werden vereinigt:
F9 (Gläser 91-102): 2.5 g angereichertes **3;**
F10 (Gläser 103-125) **3** und **4:** 6.1 g;
F11 (Gläser 126-142): **2** und **4:** 5.3 g,
F12 (Gläser 143-148): **2** und **4:** 1.5 g.
Zur weiteren Aufreinigung werden die Fraktionen portionsweise mittels wiederholter präparativer RP HPLC aufgetrennt (Säule: Synergi Hydro RP, d = 2,5 cm, l = 25 cm; Laufmittel: isokratisch MeOH/MeCN/H2O = 4/14/82; UV-Detektion: 254 nm; Fluss: 35 ml/Min.): 0,24 g (0,05%) 3; 0,45 g (0,09%) **4** und 0,08 g (0,02%) **2.**

**Tabelle 1: NMR-Daten zu 2**

| # | Zuckereinheit | δ ¹H Multiplizität J [Hz] | δ ¹³C | HMBC H# -> C# | ROESY H# -> H# |
|---|---|---|---|---|---|
| 2 | | | 158,2 | | |
| 3 | | | 134,7 | | |
| 4 | | | 179,2 | | |
| 5 | | | 162,8 | | |
| 6 | | 6,22 d 2.0 | 99,6 | C-7, C-5, C-10, C-8 | |
| 7 | | | 165,6 | | |
| 8 | | 6,43 d 2.0 | 94,5 | C-7, C-9, C-10, C-6 | |
| 9 | | | 158,1 | | |
| 10 | | | 105,5 | | |
| 1' | | | 122,9 | | |
| 2' | | 7,98 d 2.0 | 114,2 | C-2, C-3', C-4', C-6' | OMe |
| 3' | | | 148,2 | | |
| 4' | | | 150,9 | | |
| 5' | | 6,94 d 8.5 | 115,8 | C-4', C-3', C-1' | H-6' |
| 6' | | 7,62 dd 2.0, 8.5 | 123,8 | C-2, C-4', C-2' | H-5' |
| OMe | | 4.00 s | | C-3' | |
| 1" | β-D-Gal | 5,51 d 7.8 | 101,1 | C-5", **C-3** | H-3", H-4", H-5", H-6"a, H-6"b |
| 2" | β-D-Gal | 4,05 dd 7.8, 9.5 | **79,1** | C-1"', C-1", C-3" | **H-1"',** |
| 3" | β-D-Gal | 3,77 dd 3.3, 9.5 | 74,9 | C-2" | H-1", H-6"b, |
| 4" | β-D-Gal | 3,82 dd 1.1, 3.3 | 69,9 | C-3", C-2" | H-1", H-1"', H-4"' |
| 5" | β-D-Gal | 3,68 ddd 1.1, 5.7, 6.8 | 75,1 | C-6", C-4", C-1" | H-1", **H-1"",** |
| 6" | β-D-Gal | a) 3,75 dd 5.7, 10.2 b) 3,49 dd 6.8, 10.2 | **66,9** | C-2" (Artefakt) C-1"", C-5" | H-1", H-1"", H-6"b H-1", H-1"", H-6"a |
| 1"' | β-D-Xyl | 4,83 d 6.7 | 104,6 | C-3"', **C-2",** | H-5"'b, **H-2"** |
| 2"' | β-D-Xyl | 3,426 dd 6.7, 8.2 | 74,1 | C-3"', | H-5"'a |
| 3'" | β-D-Xyl | 3,413 dd 8.6, 8.2 | 76,3 | C-2'" | H-1"' |
| 4"' | β-D-Xyl | 3,51 ddd 8.9, 8.6, 4.7 | 70,7 | | H-5" |
| 5"' | β-D-Xyl | a) 3,98 dd 4.7, 11.8 b) 3,25 dd 8.9, 11.8 | 66,0 | C-3"', C-1"', C-4"', C-1'" | H-2"', H-5"'b H-5"'a, H-1"' |
| 1"" | a-L-Rha | 4,56 d 1.7 | 101,6 | C-6", C-5"", C-3"" | H-5", H-6"a, H-6"b, H-2"", |
| 2"" | a-L-Rha | 3,60 dd 1.7, 3.3 | 71,8 | C-4"", C-3"" | H-1"" |
| 3"" | a-L-Rha | 3,52 dd 3.3, 9.5 | 72,0 | C-4"" | |
| 4"" | a-L-Rha | 3,29 t 9.5 | 73,6 | C-5"", C-3"", C-6"" | H-6"" |
| 5"" | a-L-Rha | 3,55 dq 6.3, 9.5 | 69,4 | C-4"" | H-6"", H-6"a |
| 6"" | a-L-Rha | 1.20 d 6,2 | 17.8 | C-5"", C-4"" | H-4"", H-5"" |

### Beispiel 3: Wirksamkeit der Isorhamnetintriglycoside 2, 3 und 4 an Mäusen

Die aktivitätssteigernde Wirkung der Isorhamnetintriglycoside **2, 3** und **4** wurde an Versuchstieren im sogenannten "Hell-Dunkel-Box" System getestet.

Das Prinzip des verwendeten Testsystems beruht darauf, dass Mäuse einem Verhaltensparadigma ausgesetzt werden, das zwei gegensätzliche Verhaltensmuster aktiviert. Die Box besteht aus zwei Kompartimenten von denen ein kleinerer dunkler Teil von einem hellen und größeren Teil durch eine Wand getrennt wird. In der Mitte der Wand befindet sich eine kleine Öffnung, die es dem Versuchstier ermöglicht zwischen den beiden Kompartimenten zu wechseln. Der Versuch beginnt mit dem Einsetzen der Maus in das helle Feld. Das angeborene Dunkelpräferenzverhalten führt dazu, dass die Tiere nach einer kurzen Latenzzeit in das dunkle -sichere Kompartiment wechseln. Nach kurzer Zeit führt aber das ebenso stark ausgeprägte Neugierverhalten (Erkundungsverhalten) dazu, dass die Mäuse wieder in das helle Kompartiment wechseln. Somit stehen zwei angeborene Verhaltensweisen, nämlich die Dunkelpräferenz (Vermutlich als Schutz vor Beutegreifern) und das Erkundungsverhalten (wichtig für Nahrungssuche, Partnersuche) im Konflikt, die für eine Dauer von 3 Minuten aufgezeichnet werden. Arzneimittel, die beruhigende pharmakologische Effekte haben, führen zu einer Verminderung der Wechselhäufigkeit zwischen den Kompartimenten, während Arzneimittel mit stimulierenden Aktivitäten die Wechselhäufigkeit steigern. Die Wechselhäufigkeit ist somit ein Index für aktivitätsgesteuertes Erkundungsverhalten als Zeichen einer Habituation (M. Bourin, and M. Hascoet, "The mouse light/dark box test", Eur. J. Pharmacol. 463 (2003) 55 - 65). Die Versuchsanordnung betrachtet somit Verhaltensparameter, die geeignet sind das Maß an Aktivität zu messen und zu bewerten.

Um die aktivitätssteigernden Effekte der Isorhamnetintriglycoside **2, 3** und **4** zu testen wurden Mäuse einmalig mit verschieden hohen Dosierungen der Flavone behandelt. Zum Vergleich wurden einige Tiere entweder mit dem sedierenden Benzodiazepin Lorazepam oder mit dem aktivitätssteigernden Präparat Methamphetamin oder mit einer wirkungslosen Kontrolllösung behandelt. Nach 60 Minuten wurden die Tiere in das helle Kompartiment der "Hell-Dunkel-Box" gesetzt und über einen Zeitraum von 3 Minuten beobachtet. Als Maß der Aktivität wurde die Häufigkeit der Feldwechsel gemessen. Orale, einmalige Applikation von Isorhamnetin-triglycosid **2** in einer Dosierung von 3 und 10 mg/kg führt zu einer deutlichen, signifikanten Steigerung der Wechselhäufigkeit in der "Hell-Dunkel-Box". Bei einer Applikation von 30 mg/kg geht dieser Effekt verloren (ähnlich wie bei dem Standard Stimulanz "Methamphetamin", Tab. 3).

**Tabelle 2: Wirkung der Isorhamnetintriglycoside 2 und 3 im Vergleich zum Anxiolytikum Lorazepam. Gemessen wird die Anzahl der Feldwechsel im Vergleich zur Vehikel-Kontrolle.**

| Substanz | **2** % der Kontrolle | **3** % der Kontrolle | Lorazepam % der Kontrolle |
|---|---|---|---|
| 0,3 mg/kg po | | | -46 |
| 3 mg/kg po | + 92 | +15 | |
| 10 mg/kg po | +100 # | + 54 # | |
| 30 mg/kg po | +38 | + 52 # | |

| | | | |
|---|---|---|---|
| # Irrtumswahrscheinlichkeit p < 0,05 versus Kontrolle, po = peroral | | | |

**Tabelle 3: Vergleichssubstanz Methamphetamin:**

| | | | |
|---|---|---|---|
| Methamphetamin zeigt eine deutliche und dosisabhängige Steigerung der Wechselhäufigkeit. In der mittleren Dosierung (3,0 mg/kg) war keine Steigerung des Effektes mehr möglich, da die Versuchstiere durch die Nebenwirkungen der Substanz in ihrem Verhalten deutlich gestört waren und in der höchsten geprüften Dosierung (10 mg/kg) waren die Nebenwirkungen so stark, dass die Versuchstiere die Kompartimente nicht mehr wechselten. | | | |

| **Substanz** | **Dosis** mg/kg peroral | **Wechsel** Anzahl | **Wechsel** Prozent der Kontrolle |
|---|---|---|---|
| Kontrolle | | 7 ± 1,9 | 0 |
| Methamphetamin | 0,3 | 7,5 ± 2,1 | +7% |
| Methamphetamin | 1,0 | 10,6 ± 1,6 ## | +51% |
| Methamphetamin | 3,0 | 10,9 ± 3,3 # | +56% |
| Methamphetamin | 10,0 | 0,6 ± 1,2 ## | -91 % |

| | | | |
|---|---|---|---|
| # Irrtumswahrscheinlichkeit p < 0,05 versus Kontrolle ## Irrtumswahrscheinlichkeit p < 0,001 versus Kontrolle | | | |

### Beispiel 4: Wirksamkeit der Isorhamnetintriglycoside 2 und 4 an Ratten-Hirnschnitt-Präparaten

Um den Einfluss der Isorhamnetintriglycoside **2** und **4** auf mentale Funktionen zu testen wurde die Induktion der sogenannten Langzeitpotenzierung (LTP) verwendet. In Hirnschnitt-Präparaten von Wirbeltieren dient LTP als zelluläres Modell für Lernvorgänge, vor allem im Arbeitsgedächtnis (T. V. P. Bliss, and G. L. Collingridge, "A synaptic model of memory: Long-term potentiation in the hippocampus", Nature 361 (1993) 31 - 39). Durch wiederholte Reizung von Neuronen wird eine langandauernde Verstärkung der synaptischen Übertragung induziert, die durch eine Stabilisierung oder Aktivierung sogenannter ruhender Synapsen ("silent synapses) zu einer Neubildung von Verschaltungsmustern zustande kommt (Lernen). Die am besten untersuchte Region des Gehirns für LTP Messungen ist der Hippocampus, der für die Verarbeitung von Lernvorgängen eine zentrale Rolle spielt, allerdings findet LTP auch in anderen Hirnregionen statt (G. A. Kerchner, and R.A. Nicoll, "Silent synapses and the emergence of a postsynaptic mechanism for LTP", Nat. Rev. Neurosci. 9 (2008) 813 - 825). Bei Ratten und anderen Nagern ist die Langzeitpotenzierung insbesondere mit der Ausbildung des räumlichen Lernens assoziiert (J. R. Whitlock, A. J. Heynen, M. G. Shuler, and M. F. Bear, "Learning induced long-term potentiation in the hippocampus", Science 313 (2006) 1093 - 1097).

Die Experimente wurden an Hippocampus Schnittpräparaten von 8-12 Wochen alten weiblichen Wistar-Ratten durchgeführt. Die Hirnschnitte werden bei 35°C in einer Messkammer gehalten und mit einer Nährlösung perfundiert. Glasmikroelektroden werden im dendritischen Bereich des Stratum pyramidale (CA1) platziert um "Population Spikes" (POP-Spikes) zu registrieren. Die Schaffer-Kollateralen werden mit bipolaren Reizelektroden aus Platin-Draht stimuliert und die resultierenden Aktionspotentiale (POP Spikes) gemessen. Der eigentliche Versuch beginnt nach einer Äquilibrierungszeit von 60 Minuten in welcher die Hirnschnitte nicht manipuliert werden. Anschließend werden die Elektroden platziert und begonnen die Schaffer Kollateralen mit steigender Stromstärke zu stimulieren. Nach Erreichen der maximalen Reizantwort wird die Stromstärke so reduziert, dass nur noch 50% der maximal möglichen Reizstärke erreicht werden. Dadurch kann im späteren Versuch überprüft werden ob eine Verstärkung der Reizantwort möglich ist. Anschließend wird alle 10 Sekunden mit der submaximalen Reizstärke stimuliert und die POP Spikes registriert. Die erste LTP-Induktion beginnt mit einer repetitiven Reizung (100 Hz für 1 Sekunde). Der 1. Stimulation folgt die erneute Einzelstimulation (alle 10 Sekunden ein submaximaler Reiz) für zehn Minuten. Diese erste Stimulation dient der Überprüfung, ob der Hirnschnitt eine Reizantwort auf die LTP-Induktion zeigt. Nach Ablauf der ersten Zehn Minuten wird mit dem Substanzzulauf begonnen und weitere zehn Minuten die Größe der POP Spikes registriert. Anschließend wird alle zwanzig Minuten eine neue LTP-Induktion mit jeweils 20 Minuten andauender Registrierung durchgeführt (insgesamt 5 Induktionen). Die Prüfsubstanz bzw. die Kontrolllösung erfolgt kontinuierlich ab der 10. Minute der 1. LTP-Induktion.

Die Isorhamnetintriglycoside **2** und **4** wurden im Vergleich zu dem verwendeten Lösungsmittel getestet. Die Isorhamnetintriglycoside **2** und **4** zeigten eine deutliche Steigerung der Reizantwort nach LTP-Stimulation. **Abb. 2** zeigt den Einfluss von Isorhamnetintriglycosid **2** bzw. Kontrolllösung auf die Größe der POP Spikes nach wiederholter LTP-Induktion. Substanzzulauf beginnt 10 Minuten nach der 1. LTP-Induktion. **Abb. 3** zeigt den Einfluss von Isorhamnetintriglycosid 4 bzw. Kontrolllösung auf die Größe der POP Spikes nach wiederholter LTP-Induktion. Substanzzulauf beginnt 10 Minuten nach der 1. LTP-Induktion.

### Beispiel 5: Wirksamkeit des Extraktes gemäß Beispiel 1 an Ratten

Um den Einfluss des Opuntienextraktes gemäß Beispiel 1 auf mentale Funktionen zu testen, wurden Ratten mit dem Extrakt behandelt und im sogenannten "Morris Watermaze" die Gedächtnisleistung überprüft. Dazu wurden Ratten 6 Tage lang entweder mit dem Opuntienextrakt, oder zur Kontrolle nur mit der Trägerlösung oral behandelt. An den Versuchstagen 7, 8 und 9 wurden die Tiere in gleicher Weise behandelt, wurden aber zusätzlich nach der Behandlung in ein mit Wasser gefülltes Schwimmbecken gesetzt, wo die Tiere eine unter der Wasseroberfläche positionierte Plattform finden sollten.

Um die Beeinflussung mentaler Funktionen zu messen, wurden Ratten mit der anticholinergen Substanz Scopolamin behandelt und der Einfluß einer Vehikelbehandlung bzw. einer Extraktbehandlung auf die Scopolaminwirkung untersucht. Scopolamin wird in neurologischen Untersuchungen von kognitiven Effekten häufig verwendet, da es Aktivitäten von Temporallappen-Strukturen hemmt, die für das räumliche Lernen von Bedeutung sind. Im Morrismaze Modell führt die Scopolaminapplikation zu einer deutlichen Reduktion des räumlichen Lernens, was sich an einem vermindertem Auffinden der unter der Wasseroberfläche versteckten Insel führt. Es wurde getestet, ob eine orale Behandlung mit Opuntienextrakt diese Scopolamineffekte antagonisieren kann.

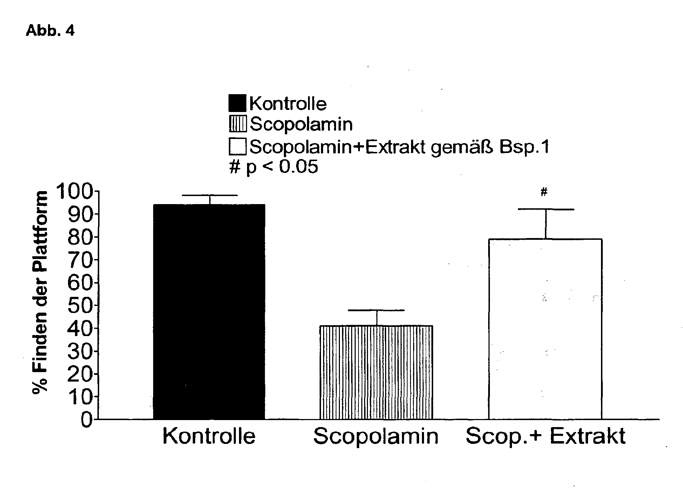

Der Opuntienextrakt zeigt eine deutliche Antagonisierung der durch Scopolamingabe ausgelösten Lernminderung **(****Abb. 4****).**

**Abb. 4****:** Die Versuchstiere wurden in zwei unabhängigen Versuchen über einen Zeitraum von 9 Tagen mit dem Opuntienextrakt gemäß Beispiel 1 behandelt (500 mg/kg/Tag po). An den letzten drei Tagen wurden die Tiere zusätzlich mit Scopolamin, oder der als Trägerlösung verwendeten physiologischen Kochsalzlösung subkutan behandelt und im Morris Maze Model auf ihre Lernleistung getestet.

Dargestellt ist die Häufigkeit des Findens der Plattform in Prozent an Tag 9 des Versuches.

Die mit Scopolamin behandelten Tiere finden im Vergleich zur Kontrolle deutlich seltener die unter der Wasseroberfläche versteckte Plattform. Werden die Tiere zusätzlich mit Opuntienextrakt behandelt finden wieder deutlich mehr Tiere die Plattform.

## Patentansprüche

1. Verwendung von Isorhamnetintriglycosiden der allgemeinen Formel **1,** 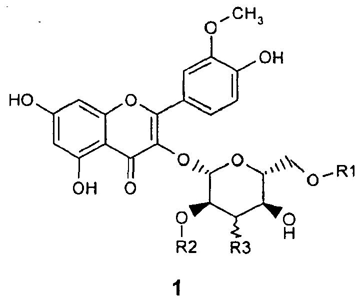
worin R1 = α-L-Rha, R2 = ß-D-Xyl, R3 = 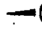 OH oder
worin R1 = α-L-Rha-(1→4)-α-L-Rha, R2 = H, R3 = 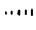 OH oder
worin R1 = α-L-Rha, R2 = ß-D-Xyl, R3 = 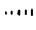 OH,
zur Herstellung eines Arzneimittels zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen,
sowie zur Verbesserung von Gedächtnisfunktionen.

2. Verwendung von Isorhamnetintriglycosiden der allgemeinen Formel 1, 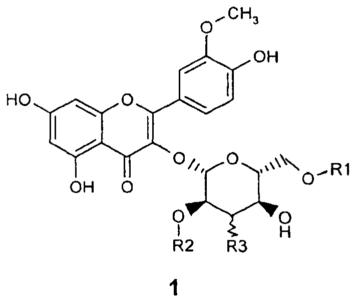
worin R1 = α-L-Rha, R2 = ß-D-Xyl, R3 = 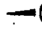 OH oder
worin R1 = α-L-Rha-(1→4)-α-L-Rha, R2 = H, R3 = 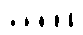 OH oder
worin R1 = α-L-Rha, R2 = ß-D-Xyl, R3 = 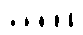 OH,
zur Herstellung eines Lebensmittels oder als Lebensmittel zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen,
sowie zur Verbesserung von Gedächtnisfunktionen.

3. Verbindung der allgemeinen Formel 1, 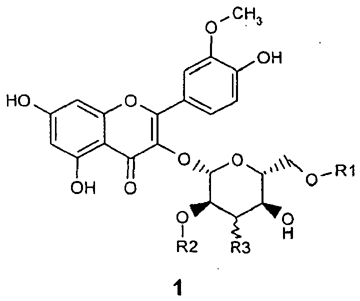
worin R1 = α-L-Rha, R2 = ß-D-Xyl, R3 = 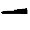 OH oder
worin R1 = α-L-Rha-(1→4)-α-L-Rha, R2 = H, R3 = 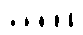 OH oder
worin R1 = α-L-Rha, R2 = ß-D-Xyl, R3 = 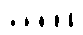 OH,
zur Verwendung als Arzneimittel zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen,
sowie zur Verbesserung von Gedächtnisfunktionen.

4. Verbindung der allgemeinen Formel **1,** 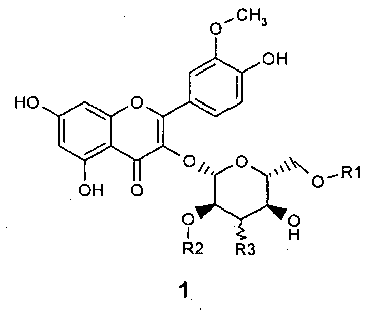
worin R1 = α-L-Rha, R2 = ß-D-Xyl, R3 = 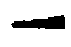 OH oder
worin R1 = α-L-Rha-(1→4)-α-L-Rha, R2 = H, R3 = 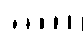 OH oder worin R1 = α-L-Rha, R2 = ß-D-Xyl, R3 = 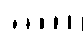 OH,
zur Verwendung als Lebensmittel zur Behandlung oder Prophylaxe von neurologischen und psychischen Erkrankungen, die mit einer Reduktion der Alltagsaktivität einhergehen und/oder mit einer Störung mentaler Funktionen verbunden sind, die die Alltagsaktivitäten beeinflussen, und/oder von Unaufmerksamkeit und Unruhezuständen, ausgewählt aus dem Aufmerksamkeits-Defizit-Hyperaktivitäts-Syndrom (ADHS), dem demenziellen Syndrom, Morbus Alzheimer, vaskuläre Demenzen, Gedächtnisstörungen anderer Ursachen,
sowie zur Verbesserung von Gedächtnisfunktionen.

5. Verwendung bzw. Verbindung zur Verwendung nach einem der Ansprüche 1 bis 4, wobei eine oder mehrere der Verbindungen der allgemeinen Formel 1 in einem Pflanzenextrakt enthalten sind.

6. Verwendung bzw. Verbindung zur Verwendung nach Anspruch 5, wobei der Pflanzenextrakt ein Extrakt aus Opuntien ist.

7. Verwendung bzw. Verbindung zur Verwendung nach Anspruch 6, wobei der Pflanzenextrakt ein Extrakt aus Blüten von Opuntia ficus-indica ist.

8. Verwendung bzw. Verbindung zur Verwendung nach Anspruch 7, wobei der Pflanzenextrakt aus Blüten von Opuntia ficus-indica mit 10 bis 90 Gew.-% Ethanol als Extraktionslösungsmittel hergestellt ist.

9. Verwendung bzw. Verbindung zur Verwendung nach Anspruch 7, wobei der Pflanzenextrakt aus Blüten von Opuntia ficus-indica mit 50 bis 70 Gew.-% Ethanol als Extraktionslösungsmittel hergestellt ist.

10. Verwendung bzw. Verbindung zur Verwendung nach einem der Ansprüche 5 bis 9, wobei die Konzentration mindestens einer der Verbindungen der allgemeinen Formel **1** im Trockenanteil des Pflanzenextraktes im Bereich von 0,01 bis 10 Gew.-% liegt.

11. Verwendung bzw. Verbindung zur Verwendung nach Anspruch 10, wobei die Konzentration mindestens einer der Verbindungen der allgemeinen Formel **1** im Trockenanteil des Pflanzenextraktes im Bereich von 0,05 bis 5% liegt.

12. Isorhamnetintriglycosid der allgemeinen Formel **1,** 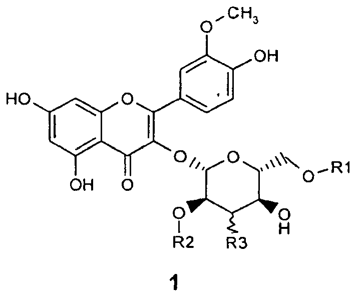 in der R1 = α-L-Rha, R2 = ß-D-Xyl und R3 = 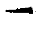 OH bedeuten.

## Claims

1. The use of isorhamnetin triglycosides of general formula **1,** 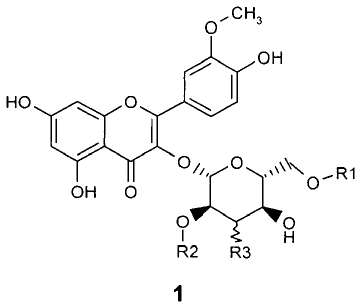
wherein R1 = α-L-rha, R2 = ß-D-xyl, R3 = 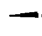 OH or
wherein R1 = α-L-rha-(1→4)-α-L-rha, R2 = H, R3 = 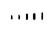 OH or wherein R1 = α-L-rha, R2 = ß-D-xyl, R3 = 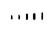 OH,
for producing a drug for treatment or prophylaxis of neurological and psychic diseases associated with reduction of everyday activity and/or connected with a disorder of mental functions influencing everyday activities and/or of attentiveness disorder and states of agitation, selected from attention deficit hyperactivity disorder (ADHD), the dementing syndrome, Alzheimer's disease, vascular dementias, memory disorders of other causes,
and for improvement of memory functions.

2. The use of isorhamnetin triglycosides of general formula **1,** 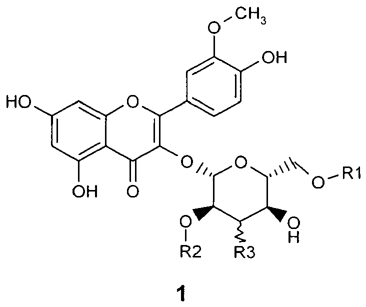
wherein R1 = α-L-rha, R2 = ß-D-xyl, R3 = 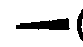 OH or
wherein R1 = α-L-rha-(1→4)-α-L-rha, R2 = H, R3 = 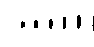 OH or
wherein R1 = α-L-rha, R2 = ß-D-xyl, R3 = 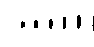 OH,
for producing food or as food for treatment or prophylaxis of neurological and psychic diseases associated with reduction of everyday activity and/or connected with a disorder of mental functions influencing everyday activities and/or of attentiveness disorder and states of agitation, selected from attention deficit hyperactivity disorder (ADHD), the dementing syndrome, Alzheimer's disease, vascular dementias, memory disorders of other causes,
and for improvement of memory functions.

3. A compound of general formula **1,** 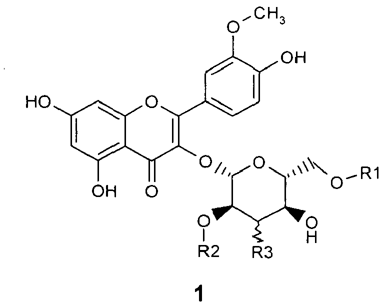
wherein R1 = α-L-rha, R2 = ß-D-xyl, R3 = 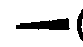 OH or
wherein R1 = α-L-rha-(1→4)-α-L-rha, R2 = H, R3 = 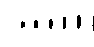 OH or
wherein R1 = α-L-rha, R2 = ß-D-xyl, R3 = 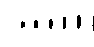 OH,
for use as drug for treatment or prophylaxis of neurological and psychic diseases associated with reduction of everyday activity and/or connected with a disorder of mental functions influencing everyday activities and/or of attentiveness disorder and states of agitation, selected from attention deficit hyperactivity disorder (ADHD), the dementing syndrome, Alzheimer's disease, vascular dementias, memory disorders of other causes,
and for improvement of memory functions.

4. The compound of general formula **1,** 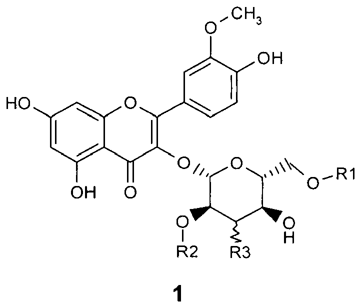
wherein R1 = α-L-rha, R2 = ß-D-xyl, R3 = 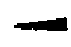 OH or
wherein R1 = α-L-rha-(1→4)-α-L-rha, R2 = H, R3 = 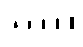 OH or
wherein R1 = α-L-rha, R2 = ß-D-xyl, R3 = 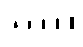 OH,
for use as food for treatment or prophylaxis of neurological and psychic diseases associated with reduction of everyday activity and/or connected with a disorder of mental functions influencing everyday activities and/or of attentiveness disorder and states of agitation, selected from attention deficit hyperactivity disorder (ADHD), the dementing syndrome, Alzheimer's disease, vascular dementias, memory disorders of other causes,
and for improvement of memory functions.

5. The use and the compound for use, respectively, according to any one of claims 1 to 4, wherein one or more of the compounds of general formula 1 are contained in a plant extract.

6. The use and the compound for use, respectively, according to claim 5, wherein the plant extract is an extract from opuntias.

7. The use and the compound for use, respectively, according to claim 6, wherein the plant extract is an extract from flowers of opuntia ficus-indica.

8. The use and the compound for use, respectively, according to claim 7, wherein the plant extract from flowers of opuntia ficus-indica is produced with 10 to 90 % by weight ethanol as extraction solvent.

9. The use and the compound for use, respectively, according to claim 7, wherein the plant extract from flowers of opuntia ficus-indica is produced with 50 to 70 % by weight ethanol as extraction solvent.

10. The use and the compound for use, respectively, according to any one of claims 5 to 9, wherein the concentration of at least one of the compounds of general formula **1** in the dry content of plant extract is in the range from 0.01 to 10 % by weight.

11. The use and the compound for use, respectively, according to claim 10, wherein the concentration of at least one of the compounds of general formula **1** in the dry content of plant extract is in the range from 0.05 to 5 %.

12. Isorhamnetin triglycoside of general formula **1,** 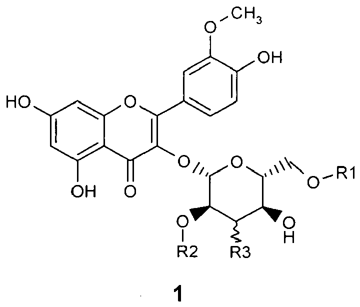 in which R1 = α-L-rha, R2 = ß-D-xyl and R3 = 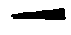 OH.

## Revendications

1. Utilisation de triglycosides d'isorhamnétine de la formule générale 1 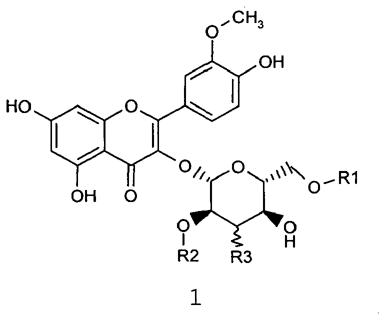
dans laquelle R1 = α-L-Rha, R2 = ß-D-Xyl, R3 = 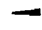 OH ou
dans laquelle R1 = α-L-Rha-(1→4)-α-L-Rha, R2=H, R3= 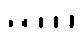 OH ou
dans laquelle R1 = α-L-Rha, R2 = ß-D-Xyl, R3 = 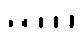 OH
pour la fabrication d'un médicament destiné au traitement ou à la prophylaxie de maladies neurologiques et psychiques qui sont accompagnées d'une réduction de l'activité quotidienne et/ou qui sont associées à un trouble des fonctions mentales qui influencent les activités quotidiennes, et/ou de l'inattention et des états d'agitation choisis parmi le trouble de déficit de l'attention avec hyperactivité (TDAH), le syndrome de démence, la maladie d'Alzheimer, les démences vasculaires, les troubles de la mémoire d'autres causes, et pour améliorer la fonction de la mémoire.

2. Utilisation de triglycosides d'isorhamnétine de la formule générale 1 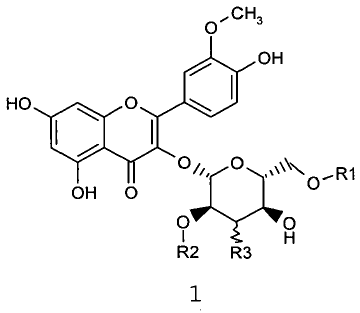
dans laquelle R1 = α-L-Rha, R2 = ß-D-Xyl, R3 = 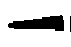 OH ou
dans laquelle R1 = α-L-Rha-(1→4)-α-L-Rha, R2=H, R3= 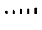 OH ou dans laquelle R1 = α-L-Rha, R2 = ß-D-Xyl, R3 = 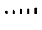 OH
pour la fabrication d'un aliment ou en tant qu'un aliment pour le traitement ou la prophylaxie de maladies neurologiques et psychiques qui sont accompagnées d'une réduction de l'activité quotidienne et/ou qui sont associées à un trouble des fonctions mentales qui influencent les activités quotidiennes, et/ou de l'inattention et des états d'agitation choisis parmi le trouble de déficit de l'attention avec hyperactivité (TDAH), le syndrome de démence, la maladie d'Alzheimer, les démences vasculaires, les troubles de la mémoire d'autres causes, et pour améliorer la fonction de la mémoire.

3. Composé de la formule générale 1 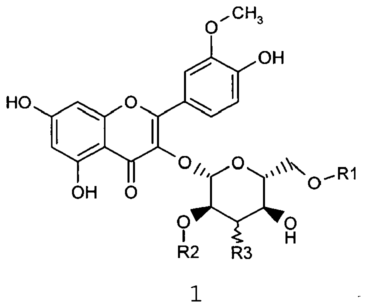
dans laquelle R1 = α-L-Rha, R2 = ß-D-Xyl, R3 = 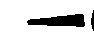 OH ou
dans laquelle R1 = α-L-Rha-(1→4)-α-L-Rha, R2=H, R3= 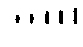 OH ou dans laquelle R1 = α-L-Rha, R2 = ß-D-Xyl, R3 = 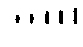 OH
pour l'utilisation comme médicament destiné au traitement ou à la prophylaxie de maladies neurologiques et psychiques qui sont accompagnées d'une réduction de l'activité quotidienne et/ou qui sont associées à un trouble des fonctions mentales qui influencent les activités quotidiennes, et/ou de l'inattention et des états d'agitation choisis parmi le trouble de déficit de l'attention avec hyperactivité (TDAH), le syndrome de démence, la maladie d'Alzheimer, les démences vasculaires, les troubles de la mémoire d'autres causes, et pour améliorer la fonction de la mémoire.

4. Composé de la formule générale 1 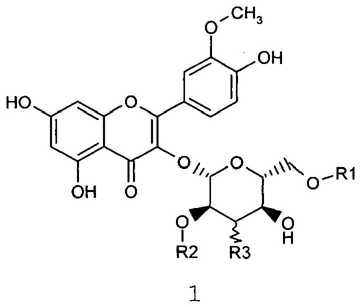
dans laquelle R1 = α-L-Rha, R2 = ß-D-Xyl, R3 = 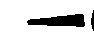 OH ou
dans laquelle R1 = α-L-Rha-(1→4)-α-L-Rha, R2=H, R3= 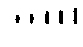 OH ou dans laquelle R1 = α-L-Rha, R2 = ß-D-Xyl, R3 = 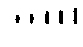 OH
pour l'utilisation comme aliment pour le traitement ou la prophylaxie de maladies neurologiques et psychiques qui sont accompagnées d'une réduction de l'activité quotidienne et/ou qui sont associées à un trouble des fonctions mentales qui influencent les activités quotidiennes, et/ou de l'inattention et des états d'agitation choisis parmi le trouble de déficit de l'attention avec hyperactivité (TDAH), le syndrome de démence, la maladie d'Alzheimer, les démences vasculaires, les troubles de la mémoire d'autres causes, et pour améliorer la fonction de la mémoire.

5. Utilisation ou composé pour l'utilisation selon l'une quelconque des revendications 1 à 4, l'un ou plusieurs des composés de la formule générale 1 étant contenu(s) dans un extrait végétal.

6. Utilisation ou composé pour l'utilisation selon la revendication 5, l'extrait végétal étant un extrait d'opuntia.

7. Utilisation ou composé pour l'utilisation selon la revendication 6, l'extrait végétal étant un extrait à partir des fleurs d'opuntia ficus-indica.

8. Utilisation ou composé pour l'utilisation selon la revendication 7, l'extrait végétal à partir des fleurs d'opuntia ficus-indica étant produit avec 10 à 90% en poids éthanol en tant que solvant d'extraction.

9. Utilisation ou composé pour l'utilisation selon la revendication 7, l'extrait végétal à partir des fleurs d'opuntia ficus-indica étant produit avec 50 à 70% en poids d'éthanol en tant que solvant d'extraction.

10. Utilisation ou composé pour l'utilisation selon l'une quelconque des revendications 5 à 9, la concentration d'au moins l'un des composés de la formule générale 1 dans la teneur en matière sèche de l'extrait végétal étant dans la gamme de 0,01 à 10% en poids.

11. Utilisation ou composé pour l'utilisation selon la revendication 10, la concentration d'au moins un des composés de la formule générale 1 dans la teneur en matière sèche de l'extrait végétal étant dans la gamme de 0,05 à 5%.

12. Triglycoside d'isorhamnétine de la formule générale 1 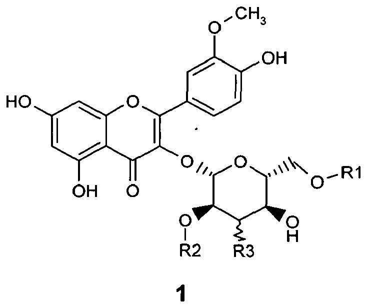 dans laquelle R1 = α-L-Rha, R2 = ß-D-Xyl et R3 = 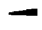 OH.
